(19) **Europäisches Patentamt / European Patent Office / Office européen des brevets**

(11) **EP 3 194 509 B1**

(12) **EUROPEAN PATENT SPECIFICATION**

(45) Date of publication and mention
of the grant of the patent:
**02.01.2019 Bulletin 2019/01**

(51) Int Cl.:
***C09D 5/14*** *(2006.01)*    ***A61L 27/30*** *(2006.01)*
***A61L 27/32*** *(2006.01)*    ***C09D 7/61*** *(2018.01)*

(21) Application number: **15793927.3**

(22) Date of filing: **15.09.2015**

(86) International application number:
**PCT/TR2015/050102**

(87) International publication number:
**WO 2016/043682 (24.03.2016 Gazette 2016/12)**

(54) **ANTIBACTERIAL NANO-SILVER COATING**

ANTIBAKTERIELLE NANOSILBERBESCHICHTUNG

REVÊTEMENT ANTIBACTÉRIEN CONTENANT DES NANOPARTICULES D'ARGENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.09.2014 TR 201410935**

(43) Date of publication of application:
**26.07.2017 Bulletin 2017/30**

(73) Proprietors:
• **Olgun, Ugursoy**
**54187 Sakarya (TR)**
• **Üstel, Fatih**
**Sakarya (TR)**

(72) Inventors:
• **Olgun, Ugursoy**
**54187 Sakarya (TR)**
• **Üstel, Fatih**
**Sakarya (TR)**

(74) Representative: **Ter Meer Steinmeister & Partner
Patentanwälte mbB
Nymphenburger Straße 4
80335 München (DE)**

(56) References cited:
**EP-A2- 2 437 798     US-A1- 2014 242 363**

**Description**

**TECHNICAL FIELD**

The present invention relates to direct coating of novel nano silver coatings, which have advanced technology antiviral, antibacterial and antimicrobial medical properties, onto ceramic, implant, etc. surfaces.

**PRIOR ART**

Microorganisms exist in the air which we respire, in our body, in the soil and on all surfaces which we contact. Bacteria lead to infection, disease, bad smell; and besides, they lead to deterioration and staining of textile, vitrified products, implant and metal products.

Because of the infection of humans as a result of the bacteria, molds, yeasts and viruses in living medium, the researches on antibacterial materials comprising various natural and inorganic substances are accelerated. Microbial smearing is one of the most important and serious problems particularly in medical devices, medicine, health services, hygienic applications, textile, food packaging, food storage, hospital and tooth surgery equipment, water purification systems. Antimicrobials bear great importance since they provide quality and reliability to pluralities of materials.

As a result, because of all of the abovementioned problems, an improvement is required in the related technical field.

**BRIEF DESCRIPTION OF THE INVENTION**

The present invention relates to antimicrobial nano-silver coating method for surfaces, for eliminating the above mentioned disadvantages and for bringing new advantages to the related technical field.

The main object of the present invention is to provide a stable nano-silver coating method at low temperatures (0-80 C).

Another object of the present invention is to provide a stable nano-silver coating method which can be rapidly applied (0-120 minutes).

Another object of the present invention is to provide a stable nano-silver coating method which can be applied homogeneously and with high efficiency.

Another object of the present invention is to provide a stable nano-silver coating method which is resistant to the deteriorations occurring on the surfaces like ceramic, metal, glass and hydroxy-apatite surfaces.

In order to realize all of the abovementioned objects and the objects which are to be deducted from the detailed description below, the present invention is a nano-silver coating method developed for providing antibacterial property to surfaces like ceramic, implant, etc. surfaces. Accordingly, the present method is characterized by comprising the steps of:

    a) Dissolving silver nitrate in pure water and mixing with acetone
    b) Adding HAP and afterwards adding PDMS to the mixture
    c) Filtering the mixture
    d) Washing the coated HAP particles with acetone and drying said HAP particles

In a preferred embodiment of the subject matter invention, steps (a) and (b) are realized at 23 °C.

In another preferred embodiment of the subject matter invention, the mixture prepared is kept for 24 hours between steps (b) and (c).

In another preferred embodiment of the subject matter invention, the prepared mixture is kept in a dark place where no air enters.

In another preferred embodiment of the subject matter invention, in step (d), the nano-silver coated hydroxy-apatite particles are dried for 2 hours in drying-oven at 105 °C.

In another preferred embodiment of the subject matter invention, after the drying process in step (d), the color of the hydroxy-apatite particles changes from light yellow to brown.

In another preferred embodiment of the subject matter invention, the particles dried in step (d) are kept inside a closed vessel in a dark place.

**BRIEF DESCRIPTION OF THE FIGURES**

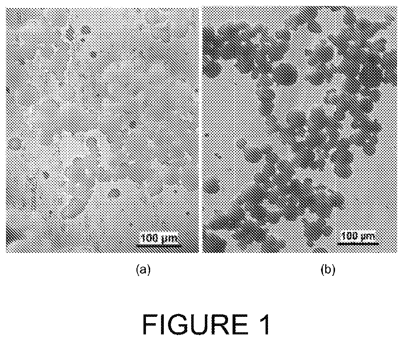

In Figure 1;

    a) Hydroxy-apatite powder light microscope image,
    b) Nano-silver doped hydroxy-apatite powder light microscope image are given.

In Figure 2, the reflection pattern of the X-rays for the Nano Ag coated HAP powder is given. (Nano Ag signal is apparently seen at 2teta=38)

In Figure 3, the HR-TEM analysis results of Nano Ag particles coated onto the HAP surface are given.

In Figure 4, the SEM EDS elemental analysis result images of the silver particles on Nano Ag-HAP surface are given.

## THE DETAILED DESCRIPTION OF THE INVENTION

In this detailed description, the subject matter nano silver coating method is explained with references to examples without forming any restrictive effect only in order to make the subject more understandable.

In said invention, the preparation of stable nano silver coatings, which are resistant to deteriorations like small particle sized oxidation and which are resistant to aggregation and breakage from the surface in time, is provided which has high efficiency and which is effective (homogeneous distribution) and rapid (0-120 minutes), and which is at low temperatures (0-80 C). The present invention can be applied by means of both submersion and the spray method. By means of the subject matter invention, the rapid nano silver coating of the ceramic powders and three dimensional implant surfaces can be provided. In the subject matter invention, as an exemplary study, nano silver coating of the hydroxy-apatite ($Ca_{10}(PO_4)_6(OH)_2$) powders is used.

First of all, in general, the hydroxy-apatite powders are coated with nano silver in 4 different proportions. Afterwards, the TEM analyses, X-Ray analyses, the EDS elemental analyses of the coated samples are realized.

In the sequencing of the metal ions for the resistance shown by the metal ions against the microorganisms, silver is the most resistant one. Among the reasons of using silver more frequently when compared with the other metals, the harmful effect of silver for human health is minimum, silver can be produced easily, and it can be found easily in nature, besides the high resistance of silver. The most frequently used component is silver nitrate (AgNO3) which can release its ions easily.

### 1. Preparation of the Nano Silver Coated Antimicrobial Hydroxy-apatite Powders by means of the Chemical Reduction Method

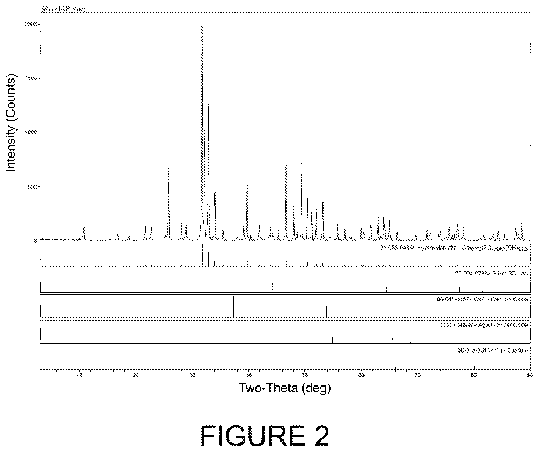

First of all, silver nitrate ($AgNO_3$) particles are dissolved in pure water, and acetone is added thereon, and the obtained mixture has been mixed in the magnetic stirrer. Hydroxy-apatite (HAP) and PDMS (polydimethylsiloxane) has been added into this mixture. The upper phase has been filtered in order to separate the nano silver coated hydroxy-apatite particles which has been settled downwards as a result of the mixing process. The particles have been washed with acetone and dried. The antibacterial activities of the produced Nano Ag coated hydroxy-apatite (AgHAP) powder have been determined. The light microscope images of the powders are given in Figure 2.

The AgHAP preparation process has been realized inside 4 different solutions comprising $AgNO_3$, HAP and PDMS at different proportions in order to compare the results and in order to find the most suitable values.

    **1.** (0.15 grams $AgNO_3$, 10 grams HAP, 0.5 ml PDMS)
    **2.** (0.3 grams $AgNO_3$, 10 grams HAP, 0.5 ml PDMS) **light color**
    **3.** (0.15 grams $AgNO_3$, 5 grams HAP, 0.5 ml PDMS) **medium dark color**
    **4.** (0.15 grams $AgNO_3$, 5 grams HAP, 2 ml PDMS) **darkest color**

The $AgNO_3$, which has been weighed by means of a precision balance, has been dissolved in 0.5 ml of pure water. 4 ml acetone has been added into the prepared silver nitrate solution, and the mixing process has been begun. Hydroxy-apatite powder has been added into this mixture. After the mixing process which lasts for 10-30 minutes, PDMS has been added and it has been rapidly mixed. After the mixing process between 5-30 minutes, 5 ml of acetone has been added and mixed. These processes have been realized at 23 °C. The solution color has been observed as yellow-brown color. It has been waited in a closed manner so as not to receive air for 24 hours in a dark place. The upper phase has been filtered in order to separate the nano silver coated hydroxy-apatite particles which have been settled down. The particles have been mixed with 20 ml acetone and washed. After the washing process, nano silver coated hydroxy-apatite particles have been dried at 105 °C in drying-oven for 2 hours. As a result of this process, it has been observed that the particles change from light yellow color to darker yellow color. It has been kept in a dark place in a closed manner.

As a result of these processes, in the mixture with no 2, the light colored nano silver coated hydroxy-apatite (HAP) powders are obtained; in the mixture with no 3, medium dark colored nano silver coated hydroxy-apatite (HAP) powders are obtained; in the mixture with no 4, the darkest colored nano silver coated hydroxy-apatite (HAP) powders are obtained.

The abovementioned substance amounts can change depending on the amount of coated final product desired to be obtained.

## 2. Antibacterial Activity Measurements of the Nano-silver Coated Hydroxy-apatite (HAP) Powders

In order to eliminate the bacteria provided on the surfaces of the samples prepared by means of coating onto the adhesive surfaced acetate, each face of the coatings has been exposed to UV (Philips Ultra Violet TUV 30w) light for 30 minutes, and has been sterilized.

1x106 CFU/ml bacteria have been sprayed onto the coatings. At T=0 moment, the sample, sprayed for determining the number of bacteria, has been rapidly washed with 10 ml 0.9 % NaCl solution. Culturing has been realized by means of the 100 $\mu$l (microliters) nutrient agar drigalski extract from the washing solution. It has been incubated for 24 hours at 37$\pm$1 °C. The colonies occurring as a result of incubation has been counted.

After the coatings, whereon bacteria are sprayed, have been closed by means of the coating film, they have been incubated for 24 hours in 96 % humidity medium at 37$\pm$1 °C. At the end of the incubation, the coatings have been washed in 0.9 % NaCl solution. 10-1 dilutions have been prepared. 100 $\mu$l of each of the washing solution and of the dilutions have been taken separately, and nutrient agar dispersion culturing has been made. The cultured nutrient media have been incubated for 24 hours at 37$\pm$1 °C. The colonies occurring at the end of the incubation have been counted. The calculation has been made according to the equation (1) given below.

$$R \% = [(B-C)/B] \times 100 \qquad (1)$$

> R: Antimicrobial effect (%)
> B: Number of bacteria in the control sample (CFU/sample)
> C: Number of bacteria in the modified sample (CFU/sample)

The determination of the antimicrobial activities of the prepared powder against Staphylococcus aureus ATCC 29213 (S. aureus) and Escherichia coli ATCC 25922 (E. Coli) has been made. It has been determined that nano-silver doped hydroxy-apatite powder has 100 % antibacterial effect against E. coli, and it has 100 % antibacterial effect against S. aureus, and it has 100 % antibacterial effect against S. Epidermis. The results have been given in Table 2a and Table 2b.

Table 2a. The colony numbers occurring in the nutrient media where direct culturing has been made for Ag-HAP (+: reproduction has been observed, -: reproduction has not been observed)

| Bacteria | HAP | Nano Ag-HAP |
|---|---|---|
| *E. coli* | + | - |
| *S. aureus* | + | - |
| *S. Epidermidis* | + | - |

Table 2b. R % values for the antibacterial activity calculated for nano Ag-HAP powder

| Bacteria | R %: Antibacterial activity | |
| | HAP | Nano Ag-HAP powder |
|---|---|---|
| *E. coli* | 0 | 100 |
| *S. aureus* | 0 | 100 |
| *S. Epidermidis* | 0 | 100 |

Bacteria reproduction has not been observed in the HAP powders coated with nano silver as can be seen in Table 2a and Table 2b. Thus, bacteria reproduction has been prevented by means of the coating process realized by means of the abovementioned method.

The X-Ray analyses of the obtained Nano Ag coated HAP powders have been made and given in Figure 2. In the X-Ray analysis application, the Nano Ag signal is apparently observed at 2teta=38.

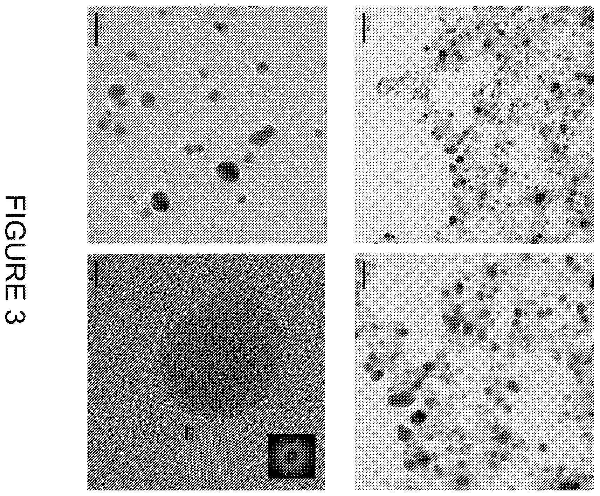

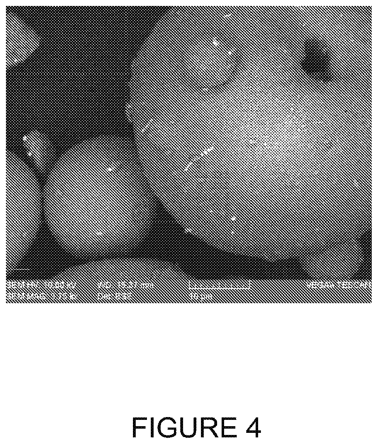

The HR-TEM analysis results of the Nano Ag particles coated onto the HAP surface are given in Figure 3, and EDS elemental analysis result images and the silver particles on the Nano Ag-HAP surface are given in Figure 4.

The protection scope of the present invention is set forth in the annexed Claims and cannot be restricted to the illustrative disclosures given above, under the detailed description. It is because a person skilled in the relevant art can obviously produce similar embodiments under the light of the foregoing disclosures, without departing from the main principles of the present invention.

**Claims**

1. A nano-silver coating method developed for providing antibacterial property to surfaces like ceramic, implant, etc. surfaces, the subject matter method is **characterized by** comprising the steps of:

   a) Dissolving silver nitrate in pure water and mixing with acetone
   b) Adding HAP and afterwards adding PDMS to the mixture
   c) Filtering the mixture
   d) Washing the coated HAP particles with acetone and drying said HAP particles

2. A nano silver coating method according to Claim 1, **characterized in that** steps (a) and (b) are realized at 23 °C.

3. A nano silver coating method according to Claim 1, **characterized in that** the mixture prepared is kept for 24 hours between steps (b) and (c).

4. A nano silver coating method according to Claim 3, **characterized in that** the prepared mixture is kept in a dark place where no air enters.

5. A nano silver coating method according to Claim 1, **characterized in that** in step (d), the nano-silver coated hydroxy-apatite particles are dried for 2 hours in drying-oven at 105 °C.

6. A nano silver coating method according to Claim 1, **characterized in that** after the drying process in step (d), the color of the hydroxy-apatite particles changes from light yellow to brown.

7. A nano silver coating method according to Claim 1, **characterized in that** the particles dried in step (d) are kept inside a closed vessel in a dark place.

**Patentansprüche**

1. Ein Nano-Silber-Beschichtungsverfahren, das entwickelt wurde, um antibakterielle Eigenschaften für die Oberflächen wie Keramik, Implantat und etc. Oberflächen bereitzustellen, wobei der Gegenstand des Verfahrens **gekennzeichnet ist, durch** die folgenden Schritte;

   a) Auflösen von Silbernitrat in gereinigtem Wasser und Mischen mit Aceton;
   b) Zugabe von HAP und anschließendes Zugegen von PDMS zur Mischung;
   c) Filtrieren der Mischung;
   d) Waschen der beschichteten HAP-Körner mit Aceton und Trocknen der HAP-Körner.

2. Nano-Silber-Beschichtungsverfahren nach Anspruch 1, **dadurch gekennzeichnet, dass** die Schritte (a) und (b) bei 23°C durchgeführt werden.

3. Nano-Silber-Beschichtungsverfahren nach Anspruch 1, **dadurch gekennzeichnet, dass** die Mischung zwischen den Schritten (b) und (c) für 24h gehalten wird.

4. Nano-Silber-Beschichtungsverfahren nach Anspruch 3 **dadurch gekennzeichnet, dass** die Mischung an einem dunklen Ort, ohne Luftzufuhr gehalten wird.

**5.** Nano-Silber-Beschichtungsverfahren nach Anspruch 1, **dadurch gekennzeichnet, dass** in Schritt (d) die Nano-Silber beschichteten Hydroxylapatit-Teilchen im Inkubator bei 105°C für 2 Stunden getrocknet werden.

**6.** Nano-Silber-Beschichtungsverfahren nach Anspruch 1, **dadurch gekennzeichnet, dass** sich die Farbe der Hydroxylapatit-Teilchen nach dem Trocknungsverfahren in Schritt (d) von hellgelb nach braun verändert.

**7.** Nano-Silber-Beschichtungsverfahren nach Anspruch 1, **dadurch gekennzeichnet, dass** die in Schritt (d) getrockneten Teilchen in einem verschlossenen Behälter unter dunklen Bedingungen gehalten werden.

## Revendications

**1.** Procédé de revêtement nano-argent mis au point pour conférer des propriétés antibactériennes à des surfaces telles que des surface céramique, des surfaces d'implant, etc., **caractérisé en ce qu'**il comprend les étapes :

   a) Dissolution du nitrate d'argent dans l'eau pure et mélange avec de l'acétone,
   b) Ajout de HAP et ensuite ajout de PDMS au mélange,
   c) Filtration du mélange.
   d) Lavage, avec de l'acétone, des particules HAP revêtues et séchage desdites particules HAP.

**2.** Procédé de revêtement nano-argent selon la revendication 1, **caractérisé en ce que** les étapes (a) et (b) sont réalisées à 23 °C.

**3.** Procédé de revêtement nano-argent selon la revendication 1, **caractérisé en ce que** le mélange préparé est laissé en attente 24 heures entre les étapes (b) et (c).

**4.** Procédé de revêtement nano-argent selon la revendication 3, **caractérisé en ce que** le mélange préparé est laissé en attente dans un endroit sombre où aucun air ne pénètre.

**5.** Procédé de revêtement nano-argent selon la revendication 1, **caractérisé en ce que** dans l'étape (d) les particules hydroxyapatite revêtues de nano-argent sont séchées pendant 2 heures dans un four de séchage à 105 °C.

**6.** Procédé de revêtement nano-argent selon la revendication 1, **caractérisé en ce qu'**après l'processus de séchage de l'étape (d), la couleur des particules hydroxyapatites passe du jaune clair au brun.

**7.** Procédé de revêtement nano-argent selon la revendication 1, **caractérisé en ce que** les particules séchées à l'étape (d) sont conservées dans un récipient fermé dans un endroit sombre.

(a)          (b)

# FIGURE 1

FIGURE 2

# FIGURE 3

FIGURE 4